# EUROPEAN PATENT APPLICATION

(11) **EP 0 537 102 A1**
(43) Date of publication of application: **14.04.1993**
(21) Application number: 92500120.8
(22) Date of filing: 23.09.1992
(51) Int. Cl.: A61L 9/04

(54) **A process for obtaining an air-freshening and cleansing product**

(30) Priority: 25.09.1991 ES 9102111
(71) Applicant: Gonzalez Hernandez, Adelaido, E-28340 Valdemoro (Madrid) (ES)
(72) Inventor: Gonzalez Hernandez, Adelaido, E-28340 Valdemoro (Madrid) (ES)
(74) Representative: Isern-Cuyas, Maria Luisa

(57) **Abstract**

The invention relates to a process for obtaining a product that acts as an air-freshener and a cleanser, achieved by placing in a mixing vessel polyol, T.D.I. (isocyanate), water, silicone, amine and scented gel in the appropriate proportions; beating this mixture for between one and a half and two and a half minutes; then adding tin octoate and beating again for as long as before and finally adding a percentage of T.D.I. (isocyanate) for the reaction thereof.

The product is then laminated and the sheets passed through a roll soaked in skin protecting soap, thereupon grouping and providing the same with the most appropriate form for marketing purposes.

## Description

As the title suggests, the invention refers to a process which yields a soap-like air-freshening and cleansing product fit to be used in personal hygiene and otherwise in connection with the cleansing of a number of objects, with no manner of limitations.

A large number of products of this kind are currently available in the market, sold under various forms of presentation, in solid, liquid or powder forms. The product of the invention acquires, once the obtention process is over, a somewhat spongy and very handy laminar form, thus allowing the same to be marketed under the most varied forms, namely slabs, strips, blocks and even rolls. The latter form can be fitted to dispensers of the kind normally used for toilet rolls and is hence very convenient to use, inasmuch as the quantity needed for each particular service can be easily detached from the rolled sheet.

The use of this product therefore poses no difficulties whatsoever, inasmuch as a small portion of the eventual form chosen for its presentation need only be taken in order for the part or object that is to be cleaned to be perfectly lathered by contact with water.

Another positive feature of this product is that having a scented gel as a constituent element, it acquires and gives off a very pleasant smell, that will be retained at all times and hence, if kept uncovered, it will also act as an efficient air-freshener.

In essence, the air-freshener cleansing product, the process for obtaining which is the subject of this patent application, comprises the following components:
Polyol, T.D.I. (isocyanate), water, silicone, amine, tin octoate and scented gel.

The process for obtaining the product is as follows:
The first stage involves placing in a suitable mixing vessel 100 parts of polyol; 4.257 parts of water (H₂O); 1.386 parts of silicone; 0.319 parts of amine and 0.610 parts of scented gel, then beating all the components for between one and a half and two and a half minutes.

The second stage involves adding to the mixture obtained from this beating 0.206 parts of tin octoate and then beating the same again, for about as long as during the first stage.

The third stage involves adding to the resultant product 57.300 parts of T.D.I. (isocyanate) for reaction thereof.

The above percentages, and the length of the various process operations can vary, within small limits, without this altering the essence of the invention.

Once the product has been obtained thus, the next stages will involve finishing and preparing the same for marketing purposes under the most varied forms.

In these stages, the product obtained is passed through laminating machines, that cause the same to be laminated in the desired sizes, and when lamination is over, the sheets are in turn passed through rollers, soaked in a skin-protecting soap, thereupon for the whole process to be completed, finally preparing the sheets under a large variety of forms, for marketing purposes.

Having described the invention in sufficient detail, it only remains to be said that all and any such circumstances as do not substantially affect the essence can be altered, all of which shall come under the protection being sought.

## Claims

**1.-** A PROCESS FOR OBTAINING AN AlR-FRESHENING AND CLEANSING PRODUCT, characterised in that the first stage involves placing in a suitable mixing vessel 100 parts of polyol; 4.257 parts of water (H₂O); 1.386 parts of silicone; 0.319 parts of amine and 0.610 parts of scented gel, and then beating all the components for between one and a half and two and a half minutes.

**2.-** A PROCESS FOR OBTAINING AN AIR-FRESHENING AND CLEANSING PRODUCT, as in claim 1, characterised in that the second stage involves adding to the mixture obtained 0.206 parts of tin octoate and then beating the product again, for about as long as during the first stage.

**3.-** A PROCESS FOR OBTAINING AN AIR-FRESHENING AND CLEANSING PRODUCT, as in claims 1 and 2, characterised in that the third stage involves adding to the resultant product 57.300 parts of T.D.I. (isocyante) for reaction thereof.

**4.-** A PROCESS FOR OBTAINING AN AIR-FRESHENING AND CLEANSING PRODUCT, as in claims 1 to 3, characterised in that the next stage involves passing the: product obtained through laminating machines, that cause the same to be laminated in the desired sizes, and the sheets obtained are then passed through rollers soaked in a skin-protecting soap, thereupon providing the same with the most appropriate and desired form for marketing purposes.
